# EUROPEAN PATENT APPLICATION

(11) **EP 4 567 678 A1**
(43) Date of publication of application: **11.06.2025**
(21) Application number: 23849982.6
(22) Date of filing: 26.07.2023
(51) Int. Cl.: G06N 3/08, A61B 5/372, G06N 3/004

(54) **BRAIN RESPONSE SPACE GENERATION DEVICE, EVALUATION DEVICE, AND BRAIN RESPONSE SPACE GENERATION METHOD**

(30) Priority: 01.08.2022 JP 2022122642
(71) Applicant: National Institute of Information and Communications Technology, Koganei-shi, Tokyo 184-8795 (JP)
(72) Inventor: NISHIDA Satoshi, Koganei-shi, Tokyo 184-8795 (JP)
(74) Representative: Hautier IP - MC/EP
(86) International application number: PCT/JP2023/027417
(87) International publication number: WO 2024/029427

(57) **Abstract**

A brain response space generation device includes: a feature volume extracting unit configured to extract a feature volume of modality data for stimulation associated with each of two or more types of modalities of senses and language; and a brain response space generating unit configured to generate a brain response space on the basis of a measurement result of brain activities in response to the modality data given to a test subject by a brain activity measuring unit and the feature volume extracted by the feature volume extracting unit. The brain response space which indicates a representation space of a brain response to the modality data and is shared by the two or more types of modalities.

## Description

### TECHNICAL FIELD

The present invention relates to a brain response space generation device, an evaluation device, and a brain response space generation method.

Priority is claimed on Japanese Patent Application No. 2022-122642, filed August 1, 2022, the content of which is incorporated herein by reference.

### BACKGROUND ART

Recently, technology of predicting a brain response pattern which is generated in response to inputting of arbitrary information has become known (for example, see Patent Document 1). In the technology described in Patent Document 1, comparison between appealing details of audiovisual content and details actually felt by a user is performed using a predicted value of a brain response pattern to a language label added to the audiovisual content, and the comparison result is used, for example, to evaluate the audiovisual content.

### Citation list

### Patent Document

Patent Document 1: Japanese Unexamined Patent Application, First Publication No. 2017-129924

### SUMMARY OF THE INVENTION

### Technical Problem

However, in the related art described in Patent Document 1, only language information has been handled in a brain response space. Accordingly, in the related art, it is not possible to cope with a plurality of modalities (sense types) indicating senses such as a visual sense, an auditory sense, a tactile sense, an olfactory sense, and a sense of taste or language information, and it is difficult to apply to information of a plurality of modalities (multimodal information).

The present invention was made to solve the aforementioned problem, and an objective thereof is to provide a brain response space generation device, an evaluation device, and a brain response space generation method that can evaluate a brain response corresponding to multimodal information.

### Solution to Problem

In order to achieve the aforementioned objective, according to an aspect of the present invention, there is provided a brain response space generation device including: a feature volume extracting unit configured to extract a feature volume of modality data for stimulation associated with each of two or more types of modalities of senses and language; and a brain response space generating unit configured to generate a brain response space on the basis of a measurement result of brain activities in response to the modality data given to a test subject by a brain activity measuring unit and the feature volume extracted by the feature volume extracting unit. The brain response space indicates a representation space of a brain response to the modality data and is shared by the two or more types of modalities.

In the brain response space generation device according to the aspect of the present invention, the brain response space generating unit may generate the brain response space on the basis of brain response data which is the measurement result through machine learning and generate a prediction model for predicting a representation of the brain response space from the feature volume such that an error between representation data of the brain response space based on the brain response data which is the measurement result and representation data of the brain response space based on a brain response pattern predicted from the feature volume is minimized.

In the brain response space generation device according to the aspect of the present invention, the prediction model may include a unimodal part configured to input the feature volume for each modality and a multimodal part configured to convert an output of the unimodal part to a representation of the brain response space, which use a deep neural network, and the brain response space generating unit may optimize parameters of the deep neural network in the unimodal part and the multimodal part through learning and generate the prediction model.

In the brain response space generation device according to the aspect of the present invention, the brain response space generating unit may reduce the number of dimensions of the brain response space on the basis of the measurement results of a plurality of test subjects.

According to another aspect of the present invention, there is provided an evaluation device including an estimation processing unit configured to estimate a position corresponding to the modality data to be evaluated in the brain response space from the modality data to be evaluated on the basis of the brain response space generated by the aforementioned brain response space generation device.

According to another aspect of the present invention, there is provided a brain response space generation method including: a brain activity measuring step of causing a brain activity measuring unit to give modality data for stimulation associated with each of two or more types of modalities of senses and language to a test subject and to measure a brain activity; a feature volume extracting step of causing a feature volume extracting unit to extract a feature volume of the modality data; and a brain response space generating step of causing a brain response space generating unit to generate a brain response space on the basis of a measurement result of the brain activity in response to the modality data given to the test subject in the brain activity measuring step and the feature volume extracted in the feature volume extracting step. The brain response space indicates a representation space of a brain response to the modality data and is shared by the two or more types of modalities.

### Advantageous Effects of Invention

According to the present invention, it is possible to evaluate a brain response corresponding to multimodal information.

### BRIEF DESCRIPTION OF DRAWINGS

[FIG. 1] A functional block diagram showing an example of an evaluation system according to an embodiment.
[FIG. 2] A diagram schematically showing a process of generating a brain response space according to the embodiment.
[FIG. 3] A diagram showing an example of a prediction model using a deep neural network according to the embodiment.
[FIG. 4] A flowchart showing an example of operations in the evaluation system according to the embodiment.
[FIG. 5] A flowchart showing an example of an evaluation process that is performed by the evaluation device according to the embodiment.

### DESCRIPTION OF EMBODIMENTS

Hereinafter, a brain response space generation device, an evaluation device, and a brain response space generation method according to an embodiment of the present invention will be described with reference to the accompanying drawings.

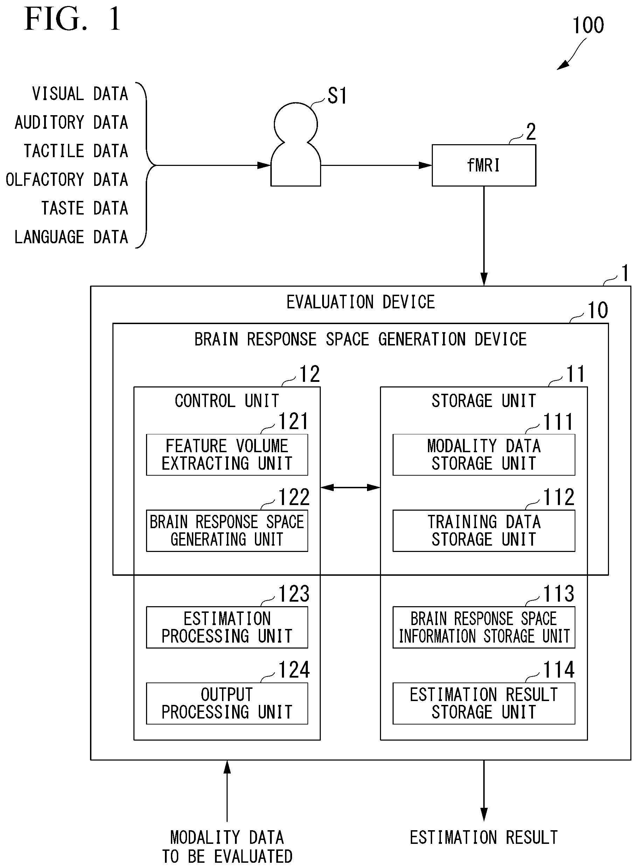

FIG. 1 is a functional block diagram showing an example of an evaluation system 100 according to an embodiment.

As shown in FIG. 1, the evaluation system 100 includes an evaluation device 1 and a functional magnetic resonance imaging device (fMRI) 2.

The evaluation device 1 according to the present embodiment generates a brain response space which is shared by a plurality of types of modalities through machine learning on the basis of modality data for stimulation associated with the plurality of types of modalities and brain response data measured by giving the modality data to a test subject S1 and evaluates new modality data to be evaluated using the brain response space.

Here, a modality is a sense of a sensory organ such as a visual sense, an auditory sense, a tactile sense, an olfactory sense, or a sense of taste or perception of language and is also referred to as a sense type. **In** the present embodiment, an example in which five types of modalities including a visual sense, an auditory sense, a tactile sense, an olfactory sense, a sense of taste, and a language are used will be described.

Use of a plurality of types (at least two types) of modalities is referred to as multimodal.

Modality data is data for stimulation associated with a modality. The modality data includes, for example, visual data, auditory data, tactile data, olfactory data, taste data, and language data. Visual data is, for example, video data or image data, auditory data is, for example, sound data. Tactile data is, for example, pressure data, and olfactory data is, for example, smell data. Taste data is, for example, data associated with taste, and language data is, for example, speech data of a conversation or sentence data.

The fMRI 2 (an example of a brain activity measuring unit) measures brain activities by giving a plurality of types of modality data (training modality data) to a test subject S1. The fMRI 2 measures a brain activity of a test subject S1 in response to stimulation as brain response data by giving stimulation based on modality data to the test subject S1. The fMRI 2 outputs an fMRI signal (a brain activity signal) for visualizing a hemodynamic reaction associated with the brain activity of the test subject S1. The fMRI 2 measures a brain activity of the test subject S1 at predetermined time intervals (for example, at intervals of 2 seconds) as a representation (a raw brain response pattern) using units of measurement (for example, voxels in case of the functional MRI) and outputs the measurement result as an fMRI signal to the evaluation device 1.

The evaluation device 1 is, for example, a computer device such as a server device or a personal computer. The evaluation device 1 includes a storage unit 11 and a control unit 12.

The storage unit 11 stores various types of information used for various processes which are performed by the evaluation device 1. The storage unit 11 includes a modality data storage unit 111, a training data storage unit 112, a brain response space information storage unit 113, and an estimation result storage unit 114.

The modality data storage unit 111 stores training modality data in advance.

The training data storage unit 112 stores training data for machine learning for generating a brain response space. The training data storage unit 112 stores, for example, information indicating a type of a modality, a feature volume extracted from modality data, and a measurement result (a brain response pattern) measured by the fMRI 2 in correlation.

The brain response space information storage unit 113 stores information indicating a brain response space which is a learning result. The brain response space information storage unit 113 stores, for example, both information indicating a prediction model for predicting a brain response pattern (a representation of a brain response space) from a feature volume of modality data and information for projecting a brain response pattern to a brain response space.

The estimation result storage unit 114 stores an estimation result obtained by estimating a corresponding position of a brain response space from (a feature volume of) arbitrary modality data to be evaluated.

The control unit 12 is, for example, a processor including a central processing unit (CPU) and comprehensively controls the evaluation device 1. The control unit 12 performs various processes which are performed by the evaluation device 1. The control unit 12 includes a feature volume extracting unit 121, a brain response space generating unit 122, an estimation processing unit 123, and an output processing unit 124.

The feature volume extracting unit 121 extracts a feature volume of modality data. A feature volume of modality data includes a label, a statistic, and a sensor measurement value corresponding to each modality and a feature volume of a machine learning model.

For example, when modality data is image data or video data, the feature volume is an object label in a scene or an intermediate layer activation pattern of a convolutional neural network. For example, when modality data is speech data, the feature volume is a spectrogram or an intermediate layer activation pattern of a convolutional neural network. For example, when modality data is tactile data, the feature volume is a pressure distribution, a texture label, or the like.

For example, when modality data is olfactory data, the feature volume is a component concentration or a flavor of a chemical, a measured value of a sensor, or the like. For example, when modality data is taste data, the feature volume is a component concentration of a chemical or the like. For example, when modality data is language data, the feature volume is an embedded expression of a word or a sentence or the like.

The feature volume extracting unit 121 correlates information indicating a modality type, an extracted feature volume, and a brain response pattern (brain response data) measured by the fMRI 2 and stores the correlation result as training data in the training data storage unit 112.

The brain response space generating unit 122 generates a brain response space SP on the basis of the measurement result obtained by causing the fMRI 2 to apply modality data to a test subject S1 and measuring a brain activity thereof and the feature volume extracted by the feature volume extracting unit 121. The brain response space SP indicates a representation space of a brain response to modality data and is shared by a plurality of types (two types or more) of modalities. The brain response space generating unit 122 constructs a shared brain response space in multimodal information, for example, by performing machine learning using training data stored in the training data storage unit 112.

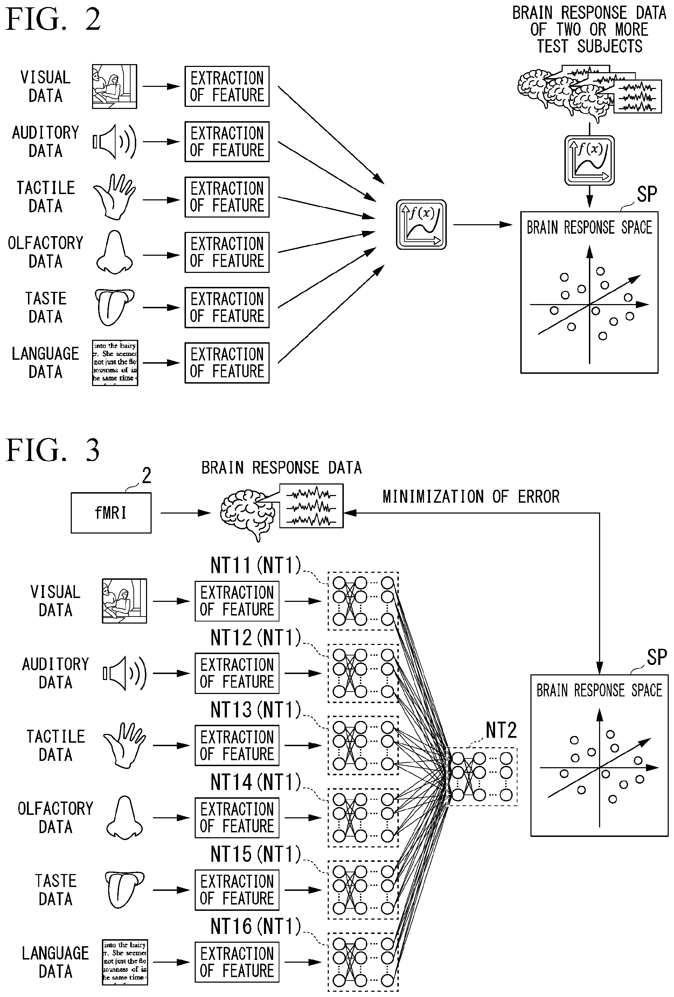

For example, the brain response space generating unit 122 generates a brain response space on the basis of brain response data which is a measurement result through machine learning and generates a prediction model for predicting a representation of the brain response space from the feature volume such that an error between representation data of the brain response space based on the brain response data which is the measurement result and representation data of the brain response space based on the brain response pattern predicted from the feature volume is minimized. A process of generating (constructing) a brain response space which is performed by the brain response space generating unit 122 will be schematically described below with reference to FIG. 2.

FIG. 2 is a diagram schematically showing a process of generating a brain response space SP according to the present embodiment.

As shown in FIG. 2, the brain response space generating unit 122 performs a process of constructing a brain response space SP and a process of constructing a prediction model.

The brain response space generating unit 122 constructs a prediction model for predicting a brain response pattern (a representation of a brain response space) by integrating and converting feature volumes extracted for each modality in the process of constructing a prediction model. The brain response space generating unit 122 constructs a brain response space SP for representing various types of modality information in a shared format in the process of constructing a brain response space SP. In the brain response space SP, input data of each modality is converted into a representation of a brain response space by the prediction model, and all the modalities are handled together.

The brain response space generating unit 122 reduces the number of dimensions of a brain response space on the basis of measurement results of a plurality of test subjects S1 at the time of construction of the brain response space SP. The brain response space generating unit 122 generates a low-dimensional representation space shared by a group from a brain response pattern, for example, using a data dimension reducing technique (for example, principal component analysis or independent component analysis) of machine learning or functional alignment (for example, a dimension reducing technique such as shared response model) for acquiring a brain data coordinate system shared by a group.

The brain response space generating unit 122 trains a model for predicting a brain response pattern (a representation of a brain response space) on the basis of data obtained by converting each modality input to a feature volume at the time of construction of the prediction model. The prediction model corresponds to a regression mode of machine learning, and training corresponds to estimating weighting parameters of a model on the basis of pair data of the feature volume and the measured brain response pattern.

Here, an arbitrary technique can be applied to a regression model, and examples thereof include linear regression, support vector regression, and regression based on deep learning. The brain response space generating unit 122 may train the prediction model for each modality or as a single model for handling all modalities together. When the prediction model is trained for each modality, a single brain response pattern needs to be predicted from one input set, and thus the brain response space generating unit 122 integrates a predicted value of the brain response pattern for each modality using a weighted average or the like.

An example in which a prediction model shared by all modalities is trained will be described below with reference to FIG. 3.

FIG. 3 is a diagram showing an example of a prediction model using a deep neural network according to the present embodiment.

FIG. 3 shows an example in which the brain response space generating unit 122 uses a prediction model using a deep neural network.

The prediction model shown in FIG. 3 includes a unimodal part NT1 for inputting data converted to a feature volume for each modality and a shared multimodal part NT2 for converting the data to a predicted value of a brain response pattern.

In this prediction model, first, data converted to a feature volume for each modality is input to the unimodal part NT1 and subjected to an hierarchical calculation process, is then input to the multimodal part NT2 and further subjected to an hierarchical process, and is output as a predicted value of the brain response pattern.

Here, a unimodal part NT11 is a unimodal part corresponding to a visual sense, a unimodal part NT12 is a unimodal part corresponding to an auditory sense, and a unimodal part NT13 is a unimodal part corresponding to a tactile sense. A unimodal part NT14 is a unimodal part corresponding to an olfactory sense, a unimodal part NT15 is a unimodal part corresponding to a sense of taste, and a unimodal part NT16 is a unimodal part corresponding to language.

In the present embodiment, the unimodal parts NT11 to NT16 are referred to as a unimodal part NT1 when they refer to an arbitrary unimodal part or when they are not particularly distinguished from each other.

The brain response space generating unit 122 individually performs a training process in the unimodal part NT13 using a brain response pattern (brain response data) corresponding to an input of each modality. The brain response space generating unit 122 performs a training process in the multimodal part NT2 as a shared part using a brain response pattern (brain response data) corresponding to an input of all modalities.

When the prediction model shown in FIG. 3 is constructed through machine learning, the brain response space generating unit 122 may receive inputs of a plurality of modalities at the same time, but all the modalities do not need to be input at the same time. The brain response space generating unit 122 uses data converted to a feature volume for each modality and corresponding brain response data to train the prediction model, performs training of parameters in the unimodal part NT1 on the basis of only an input of each corresponding modality, and performs training of parameters in the multimodal part NT2 on the basis of inputs of all the modalities.

In this way, a prediction model may include a unimodal part NT1 for inputting a feature volume for each modality and a multimodal part NT2 for converting the output of the unimodal part NT1 to a brain response pattern (a representation of a brain response space) which use a deep neural network. In this case, the brain response space generating unit 122 optimizes parameters of the deep neural network in the unimodal part NT1 and the multimodal part NT2 through learning such that an error between representation data of the brain response space of a measured value and representation data of the brain response space of a predicted value is minimized and generates the prediction model.

Referring back to FIG. 2, the brain response space generating unit 122 stores information indicating the generated prediction model and the generated brain response space SP in the brain response space information storage unit 113.

In the present embodiment, the modality data storage unit 111, the training data storage unit 112, the feature volume extracting unit 121, and the brain response space generating unit 122 correspond to the brain response space generation device 10.

The estimation processing unit 123 estimates a position corresponding to modality data to be evaluated in the brain response space SP from the modality data to be evaluated on the basis of the brain response space SP generated by the brain response space generation device 10. The estimation processing unit 123 causes the feature volume extracting unit 121 to extract a feature volume of modality data to be evaluated. The estimation processing unit 123 estimates a position in the brain response space SP from the feature volume of the modality data to be evaluated on the basis of the prediction model and the information indicating the brain response space SP stored in the brain response space information storage unit 113. The estimation processing unit 123 stores the estimation result in the estimation result storage unit 114.

The output processing unit 124 outputs the estimation result estimated by the estimation processing unit 123 to the outside. For example, the output processing unit 124 outputs position information corresponding to the modality data to be evaluated in the brain response space SP which is the estimation result stored in the estimation result storage unit 114 to the outside.

Operations of the evaluation system 100 according to the present embodiment will be described below with reference to an accompanying drawing.

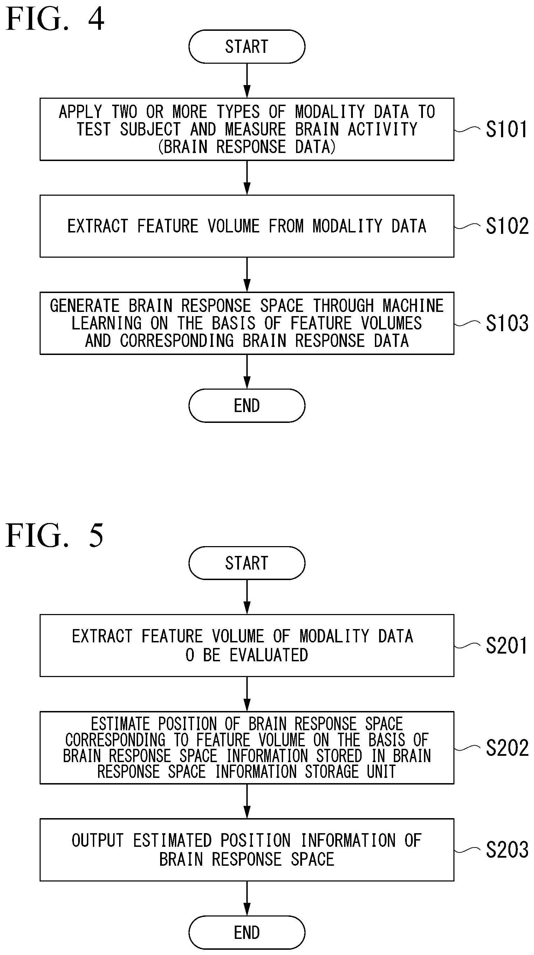

FIG. 4 is a flowchart showing an example of the operations of the evaluation system 100 according to the present embodiment.

As shown in FIG. 4, the fMRI2 applies each of a plurality of types of modality data to a test subject S1 and measures a brain activity (brain response data) (Step S101). Here, the modality data applied to the test subject S1 is the same as training data stored in the modality data storage unit 111 of the brain response space generation device 10. The fMRI 2 outputs the measured brain response data to the brain response space generation device 10.

Then, the feature volume extracting unit 121 of the brain response space generation device 10 extracts a feature volume from the modality data (Step S102). The feature volume extracting unit 121 acquires training modality data stored in the modality data storage unit 111 and extracts a feature volume. The feature volume extracting unit 121 correlates information indicating a modality type, the extracted feature volume, and the brain response pattern (brain response data) measured by the fMRI 2 and stores the correlation result as training data in the training data storage unit 112.

Then, the brain response space generating unit 122 of the brain response space generation device 10 generates a brain response space SP through machine learning on the basis of the feature volumes and the corresponding brain response data (Step S103). The brain response space generating unit 122 generates (constructs) the prediction model and the brain response space SP using the training data stored in the training data storage unit 112 as shown in FIGS. 2 and 3. The brain response space generating unit 122 stores information indicating the generated prediction model and the generated brain response space SP in the brain response space information storage unit 113. After the process of Step S103 has been performed, the brain response space generating unit 122 ends the process flow.

An evaluation process that is performed by the evaluation device 1 according to the present embodiment will be described below with reference to FIG. 5.

FIG. 5 is a flowchart showing an example of an evaluation process that is performed by the evaluation device 1 according to the present embodiment.

As shown in FIG. 5, the evaluation device 1 first extracts a feature volume to modality data to be evaluated (Step S201). That is, the feature volume extracting unit 121 of the evaluation device 1 extracts a feature volume of modality data to be evaluated.

Then, the evaluation device 1 estimates a position of the brain response space SP corresponding to the feature volume on the basis of the brain response space information stored in the brain response space information storage unit 113 (Step S202). The estimation processing unit 123 of the evaluation device 1 estimates the position in the brain response space SP from the feature volume of modality data to be evaluated on the basis of information indicating the prediction model and the brain response space SP stored in the brain response space information storage unit 113. The estimation processing unit 123 stores the estimation result in the estimation result storage unit 114.

Then, the evaluation device 1 outputs information of the estimated position in the brain response space (Step S203). For example, the output processing unit 124 of the evaluation device 1 outputs position information corresponding to the modality data to be evaluated in the brain response space SP which is the estimation result stored in the estimation result storage unit 114 to the outside. After the process of Step S203 has been performed, the output processing unit 124 ends the process flow.

As described above, the brain response space generation device 10 according to the present embodiment includes the feature volume extracting unit 121 and the brain response space generating unit 122. The feature volume extracting unit 121 extracts a feature volume of modality data for stimulation associated with each of two or more types of modalities of senses and language. The brain response space generating unit 122 generates a brain response space SP on the basis of a measurement result of brain activities in response to the modality data given to a test subject S1 from the fMRI 2 (a brain activity measuring unit) and the feature volume extracted by the feature volume extracting unit 121. The brain response space SP indicates a representation space of a brain response to the modality data and is shared by the two or more types of (a plurality of) modalities.

Accordingly, the brain response space generation device 10 according to the present embodiment can convert input data corresponding to various modalities which can be handled by the brain to a brain response representation which is shared by the modalities by using the brain response space SP which is shared by the two or more types of (a plurality of) modalities. As a result, the brain response space generation device 10 according to the present embodiment can evaluate a brain response corresponding to multimodal information.

The brain response space generation device 10 according to the present embodiment can be applied to broader problems in real societies than those in the related art by constructing a shared representation (a brain response space SP) of various types of modality information. The brain response space generation device 10 according to the present embodiment can perform numerical calculation such as comparison, combination, and conversion between data of different modalities and can be generally used as techniques for solving multimodal recognition problems of types which cannot be handled in the machine learning techniques according to the related art as well as brain information techniques according to the related art. With the brain response space generation device 10 according to the present embodiment, it is also possible to expect improvement in recognition performance based on use of characteristics of brain information, particularly, in multimodal recognition problems in which recognition or behavior of human beings.

Since the relationship between language data and various types of sense data can be quantified in a shared representation space, the brain response space generation device 10 according to the present embodiment exhibits effectiveness in multimodal search. Specifically, in a search technique according to the related art, only images, videos, and music pieces can be retrieved on the basis of keywords. With the brain response space generation device 10 according to the present embodiment, it is possible to retrieve data of a sense of taste, a tactile sense, and an olfactory sense in addition to the modalities in the related art on the basis of similarities between embedded vectors by using the generated brain response space SP. **In** the brain response space generation device 10 according to the present embodiment, since characteristics of brain information are reflected in the similarities, it is possible to expect provision of search results closer to human way of thinking. On the other hand, applying language description to sense data by estimating language data similar to a sense of taste, a tactile sense, and an olfactory sense, which it is generally difficult to convert to language, in a shared representation space is also an example of effective usage. Accordingly, with the brain response space generation device 10 according to the present embodiment, it is possible to promote communication between individuals and enhance sympathy.

For example, the brain response space generation device 10 according to the present embodiment can be used as follows by using a brain response space SP.

(Use Example 1) With the brain response space generation device 10 according to the present embodiment, for example, it is possible to retrieve corresponding Scotch Whisky using multimodal keywords such as "Scotch with tender flavor like flowers and with slight sweet aftertaste."

(Use Example 2) The brain response space generation device 10 according to the present embodiment can be used to estimate data fidelity between modalities such as estimating a degree of fidelity from, for example, an image of interior appearance in a room + music of BGM + flavor.

(Use Example 3) The brain response space generation device 10 according to the present embodiment can be used, for example, in recommendation of cross-modal products such as recommending a book preferred by a user (suitable for a user) from the user's music purchase history.

In the present embodiment, the brain response space generating unit 122 generates the brain response space SP on the basis of brain response data which is the measurement result through machine learning and generates a prediction model for predicting a representation of the brain response space from the feature volume such that an error between representation data of the brain response space based on the brain response data which is the measurement result and representation data of the brain response space based on a brain response pattern predicted from the feature volume is minimized.

Accordingly, with the brain response space generation device 10 according to the present embodiment, it is possible to appropriately estimate a corresponding position in the brain response space SP from (the feature volume) of modality data by generating the prediction model and the brain response space SP.

In the present embodiment, the prediction model includes a unimodal part NT1 configured to input the feature volume for each modality and a multimodal part NT2 configured to convert an output of the unimodal part to a brain response pattern (a representation of the brain response space), which use a deep neural network. The brain response space generating unit 122 optimizes parameters of the deep neural network in the unimodal part and the multimodal part NT2 through learning and generates the prediction model.

Accordingly, with the brain response space generation device 10 according to the present embodiment, it is possible to easily and appropriately generate a prediction model using a simple method by using a deep neural network.

In the present embodiment, the brain response space generating unit 122 reduces the number of dimensions of the brain response space on the basis of the measurement results of a plurality of test subjects S1.

Accordingly, with the brain response space generation device 10 according to the present embodiment, it is possible to reduce the number of dimensions, for example, from several tens of thousands of dimensions to several hundreds of dimensions and to acquire an effective brain response representation (the brain response space SP) with low redundancy as an information representation.

The evaluation device 1 according to the present embodiment includes the estimation processing unit 123 configured to estimate a position corresponding to the modality data to be evaluated in the brain response space SP from the modality data to be evaluated on the basis of the brain response space generated by the brain response space generation device 10.

Accordingly, with the evaluation device 1 according to the present embodiment, it is possible to achieve the same advantageous effects as in the brain response space generation device 10 and to evaluate a brain response corresponding to multimodal information. Since a position corresponding to modality data to be evaluated in the brain response space SP can be acquired from the modality data to be evaluated, the evaluation device 1 according to the present embodiment can be used, for example, in comparison of similarity between modalities or clustering modalities. The evaluation device 1 according to the present embodiment can generally use the brain response space SP as a vector expression of input data with a representation shared by a plurality of modalities.

A brain response space generation method according to the present embodiment includes a brain activity measuring step, a feature volume extracting step, and a brain response space generating step. In the brain activity measuring step, the fMRI 2 gives modality data for stimulation associated with each of two or more types of modalities of senses and language to a test subject S1 and measures a brain activity. In the feature volume extracting step, the feature volume extracting unit 121 extracts a feature volume of the modality data. In the brain response space generating step, the brain response space generating unit 122 generates a brain response space SP on the basis of a measurement result of the brain activity in response to the modality data given to the test subject S1 in the brain activity measuring step and the feature volume extracted in the feature volume extracting step. The brain response space SP indicates a representation space of a brain response to the modality data and is shared by the two or more types of modalities.

Accordingly, with the brain response space generation method according to the present embodiment, it is possible to achieve the same advantageous effects as in the brain response space generation device 10 and to evaluate a brain response corresponding to multimodal information.

The present invention is not limited to the aforementioned embodiment and can be modified without departing from the gist of the invention.

For example, in the aforementioned embodiment, the brain response space generation device 10 and the evaluation device 1 are constructed as a single device, but the present invention is not limited thereto. The brain response space generation device 10 and the evaluation device 1 may be constructed as separate devices. Some of the constituents of the brain response space generation device 10 and the evaluation device 1 may be provided outside thereof.

In the aforementioned embodiment, the fMRI 2 is used as the brain activity measuring unit, but the present invention is not limited thereto. Other brain activity measuring techniques such as electroencephalography (EEG), magneto-encephalo-graphy (MEG), electrocorticogram (ECoG), and functional near-infrared spectroscopy (fNIRS) may be used.

In the aforementioned embodiment, the prediction model includes the unimodal part NT1 and the multimodal part NT2, but the present invention is not limited to this example. Another prediction model may be used as long as it is a model that receives data converted to a feature volume of each modality as an input and outputs a predicted value of a brain response pattern (a representation of a brain response space) which is shared by the modalities. In a simpler installation example, the prediction model may be constructed as a regression model for connecting data converted to a feature volume of each modality as a vector and predicting a brain response pattern with it as an input.

In the aforementioned embodiment, a visual sense, an auditory sense, a tactile sense, an olfactory sense, a sense of taste, and language are used as modalities, but the present invention is not limited to this example. For example, a mood or an atmosphere may be used as a modality. Six types of modalities are used as a plurality of types of modalities, but the present invention is not limited to this example. A different number of types of modalities (for example, two types or three types) may be used as long as the number of types of modalities is two or more.

The constituents of the evaluation device 1 and the brain response space generation device 10 include a computer system therein. The processes in the constituents of the evaluation device 1 and the brain response space generation device 10 may be performed by recording a program for realizing the functions of the constituents of the evaluation device 1 and the brain response space generation device 10 on a computer-readable recording medium and causing a computer system to read and execute the program recorded on the recording medium. Here, "causing a computer system to read and execute a program recorded on a recording medium" includes installing the program in the computer system. The "computer system" mentioned herein includes an operating system (OS) or hardware such as peripherals.

The "computer system" may include a plurality of computer devices which are connected via a network including a communication line such as the Internet, WAN, LAN, or a dedicated line. The "computer-readable recording medium" is a portable medium such as a flexible disk, a magneto-optical disc, a ROM, or a CD-ROM or a storage device such as a hard disk incorporated into a computer system. In this way, the recording medium storing a program may be a non-transitory recording medium such as a CD-ROM.

The recording medium may include a recording medium provided inside or outside which can be accessed from a delivery server to deliver the program. A configuration in which a program is divided into a plurality of parts, the parts are downloaded at different timings, and then the downloaded parts are assembled by the constituents of the evaluation device 1 and the brain response space generation device 10 may be employed, or different delivery servers may be used to deliver the divided program parts. The "computer-readable recording medium" may include a medium that holds a program for a predetermined time such as a volatile memory (RAM) in a computer system serving as a server or a client when the program is transmitted via a network. The program may be a program for realizing some of the aforementioned functions. The program may be a program capable of realizing the aforementioned functions in combination with another program stored in advance in the computer system, a so-called differential file (a differential program).

Some or all of the functions may be realized as an integrated circuit such as a large scale integration (LSI) circuit. The functions may be individually formed as processors, or some or all thereof may be integrated as a processor. The integration technique is not limited to LSI, and the functions may be realized by a dedicated circuit or a general-purpose processor. When integration technology replacing LSI appears with development of semiconductor technology, an integrated circuit based on the technology may be used.

### REFERENCE SIGNS LIST

1 Evaluation device
2 fMRI
10 Brain response space generation device
11 Storage unit
12 Control unit
111 Modality data storage unit
112 Training data storage unit
113 Brain response space information storage unit
114 Estimation result storage unit
121 Feature volume extracting unit
122 Brain response space generating unit
123 Estimation processing unit
124 Output processing unit
NT1, NT11 to NT16 Unimodal part
NT2 Multimodal part
S1 Test subject
SP Brain response space

## Claims

1. A brain response space generation device comprising:
a feature volume extracting unit configured to extract a feature volume of modality data for stimulation associated with each of two or more types of modalities of senses and language; and
a brain response space generating unit configured to generate a brain response space on the basis of a measurement result of brain activities in response to the modality data given to a test subject by a brain activity measuring unit and the feature volume extracted by the feature volume extracting unit, the brain response space indicating a representation space of a brain response to the modality data, the brain response space being shared by the two or more types of modalities.

2. The brain response space generation device according to claim 1, wherein the brain response space generating unit generates the brain response space on the basis of brain response data which is the measurement result through machine learning and generates a prediction model for predicting a representation of the brain response space from the feature volume such that an error between representation data of the brain response space based on the brain response data which is the measurement result and representation data of the brain response space based on a brain response pattern predicted from the feature volume is minimized.

3. The brain response space generation device according to claim 2, wherein the prediction model includes a unimodal part configured to input the feature volume for each modality and a multimodal part configured to convert an output of the unimodal part to a representation of the brain response space, which use a deep neural network, and
wherein the brain response space generating unit optimizes parameters of the deep neural network in the unimodal part and the multimodal part through learning and generates the prediction model.

4. The brain response space generation device according to claim 1, wherein the brain response space generating unit reduces the number of dimensions of the brain response space on the basis of the measurement results of a plurality of test subjects.

5. An evaluation device comprising an estimation processing unit configured to estimate a position corresponding to the modality data to be evaluated in the brain response space from the modality data to be evaluated on the basis of the brain response space generated by the brain response space generation device according to any one of claims 1 to 4.

6. A brain response space generation method comprising:
a brain activity measuring step of causing a brain activity measuring unit to give modality data for stimulation associated with each of two or more types of modalities of senses and language to a test subject and to measure a brain activity;
a feature volume extracting step of causing a feature volume extracting unit to extract a feature volume of the modality data; and
a brain response space generating step of causing a brain response space generating unit to generate a brain response space on the basis of a measurement result of the brain activity in response to the modality data given to the test subject in the brain activity measuring step and the feature volume extracted in the feature volume extracting step, the brain response space indicating a representation space of a brain response to the modality data, the brain response space being shared by the two or more types of modalities.
